# EUROPEAN PATENT APPLICATION

(11) **EP 2 014 315 A1**
(43) Date of publication of application: **14.01.2009**
(21) Application number: 07736890.0
(22) Date of filing: 16.03.2007
(51) Int. Cl.: A61L 27/00, A61F 2/28

(54) **METHOD OF CONSTRUCTING ARTIFICIAL BONE**

(30) Priority: 13.04.2006 JP 2006110546
(71) Applicant: Sagawa Printing Co., Ltd., Muko-shi Kyoto (JP); Kyoto University, Kyoto-shi, Kyoto 606-8501 (JP); Chubu University Educational Foundation, Kasugai-shi, Aichi 487-8501 (JP)
(72) Inventor: NAKAMURA, Takashi, Kyoto-shi, Kyoto 606-8501 (JP); KOKUBO, Tadashi, Kasugai-shi, Aichi 487-8501 (JP); MATSUSHITA, Tomiharu, Kasugai-shi, Aichi 487-8501 (JP); FUJIBAYASHI, Shunsuke, Kyoto-shi, Kyoto 606-8501 (JP); NISHIDA, Nobukatsu, Muko-shi, Kyoto 617-8588 (JP)
(74) Representative: Hart-Davis, Jason
(86) International application number: PCT/JP2007/000233
(87) International publication number: WO 2007/122783

(57) **Abstract**

A method for producing an artificial bone precursor is disclosed. The method comprises:extracting a part corresponding to a cancellous bone and/or a cortical bone from digitalized three-dimensional image data of a living bone, followed by setting a center line on the part; drawing a beam or a wall having a uniform diameter or thickness along the center line to form artificial bone image data; and stacking a sintered layer by laser-sintering a powder of titanium and the like based on the artificial bone image data. The precursor is suitable for an artificial bone having excellent osteoconductivity and osteoinductivity.

## Description

### TECHNICAL FIELD

The present invention relates to a method for producing an artificial bone, namely a bone substitute material, and in particular, to a method for producing an artificial bone having excellent osteoconductivity and osteoinductivity, and appropriate mechanical strength.

### BACKGROUND ART

Titanium is highly resistant to corrosion in a living body and is highly biocompatible, and is therefore expected to be used an artificial bone material. An artificial bone material to be implanted in a living body is desired to be porous because of the necessity to form a living bone or bond to a surrounding living bone. It is conventionally known that a titanium porous body is obtained by pressure-molding a titanium powder, which is mixed with a pore-forming material if necessary, to obtain a molded body and then sintering the molded body (Patent Document 1). In recent years, there is also proposed a technique that a titanium powder is laser-sintered based on three-dimensional digital image data, and the sintered layer is stacked (Non-Patent Document 1).
On the other hand, as a measure of facilitating bonding between titanium and a living bone, such a technique is known that titanium is heated directly after being subjected to an alkaline treatment, or heated after dealkalization, to form a sodium titanate layer or an anatase layer on a surface of titanium (Patent Documents 2 and 3). Also known is a technique that an apatite layer is formed thereon by immersing it in a simulated body fluid subsequently as is necessary (Patent Documents 2 and 3).
Patent Document 1: JP2002-285203,A
Patent Document 2: JP2775523,B
Patent Document 3: JP2002-102330,A
Non-Patent Document 1: Biomaterials 27(2006)955-963
Non-Patent Document 2:
   http://www.nedo.go.jp/informations/koubo/171014_1/171014_1.html, attached sheet 1

### DISCLOSURE OF THE INVENTION

### PROBLEMS TO BE SOLVED BY THE INVENTION

However, since the methods disclosed in Patent Document 1 and Non-Patent Document 1 fails to control the shape and distribution of pores, even if the appearance resembles a living bone, properties such as the modulus of elasticity and the mechanical strength are completely different, and therefore the person to which the artificial bone is applied will feel quite uncomfortable.
Therefore, it is an object of the present invention to provide an artificial bone having excellent osteoconductivity and osteoinductivity and resembling a living bone in terms of appearance and properties, and an artificial bone precursor for the artificial bone.
In this description, the phenomenon that a living bone enters pores of an artificial bone and bonds with the artificial bone when the artificial bone is implanted in a site where the living bone is present, is called "osteoconductivity". On the other hand, the phenomenon that a living bone is formed in pores of an artificial bone when the artificial bone is implanted in a muscle is called "osteoinductivity".

### MEANS FOR SOLVING THE PROBLEMS

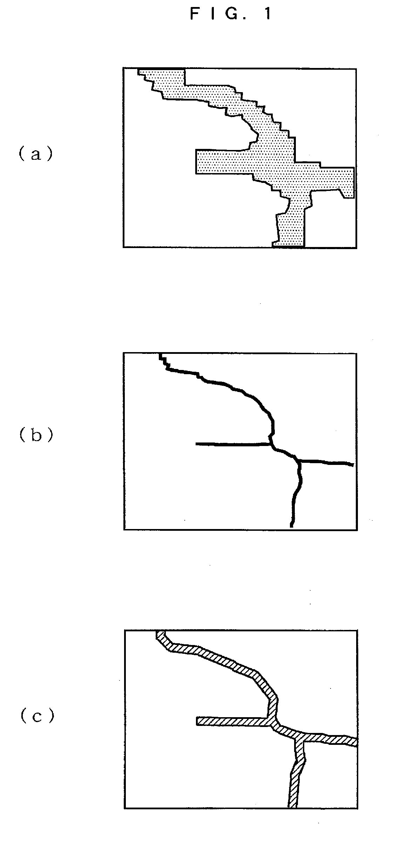

In order to solve the problems, a method for producing an artificial bone precursor of the present invention includes an extracting step, an image forming step and a modeling step as follows.
First, in digitalized three-dimensional image data of a living bone, as shown in Fig. 1(a), a part corresponding to a cancellous bone and/or a cortical bone (the hatching part in the drawing) is extracted, and a center line is set on the part as shown in Fig. 1(b) (extracting step). Next, as shown in Fig. 1(c), by drawing a beam or a wall having a uniform diameter or thickness along the center line, artificial bone image data is formed (image forming step). The beam drawn here has a shape corresponding to a cancellous bone, and the wall has a shape corresponding to a cortical bone. Thereafter, a powder of at least one material selected from metals, resins and ceramics is sintered based on the artificial bone image data using a laser system, to stack a sintered layer (modeling step).

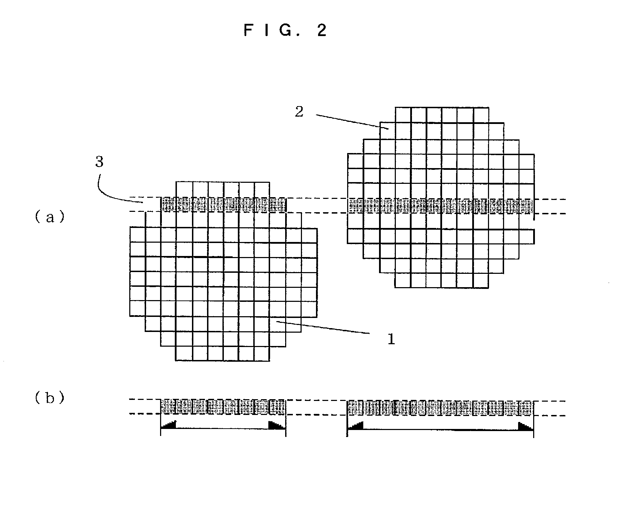

Fig. 1(a) to Fig. 1(c) depict images displayed on a monitor, and a beam and a wall actually displayed on the monitor in an image forming step seem to have much diversified diameters or thicknesses compared to those shown in Fig. 1(c). The reason of this will be explained below by taking a beam as an example. Considering the image of Fig. 1(c) as an XY plane, each beam is present not only in an unspecified position within the XY plane so as to correspond to each of the extracted cancellous bone, but also in a Z direction at different levels. That is, the image of Fig. 1(c) represents one cross section among three-dimensional image data made up of plural pieces of cross section data. Therefore, even when a beam 1 and a beam 2 have the same diameter as shown in Fig. 2 (a) as a radial cross section of beams, the diameter of the beam 1 appears to be smaller than that of the beam 2 as shown in Fig. 2 (b) when a cross section 3 is displayed on the monitor because the positions in the Z direction (above and below direction on paper) are different from each other. This phenomenon also occurs in a single beam.

According to the method of the present invention, since laser irradiation is made following a shape obtained by extracting a part corresponding to a cancellous bone and/or a cortical bone of a living bone, the resulting formed artificial bone precursor resembles a living bone not only in appearance, but also in its interior structure which is a network structure resembling a living bone. The diameter of each part in an extracted shape is not completely the same with a corresponding part in a living bone, but is even, while a cancellous bone and a cortical bone of a living bone have various diameters and thicknesses. This is because when laser irradiation is made completely following a living bone, the network is interrupted and metal particles may be missed in a too thin part because the laser doze is insufficient, while heat is conducted and sinters a peripheral part where sintering is not required because the laser doe is excess in a too thick part.

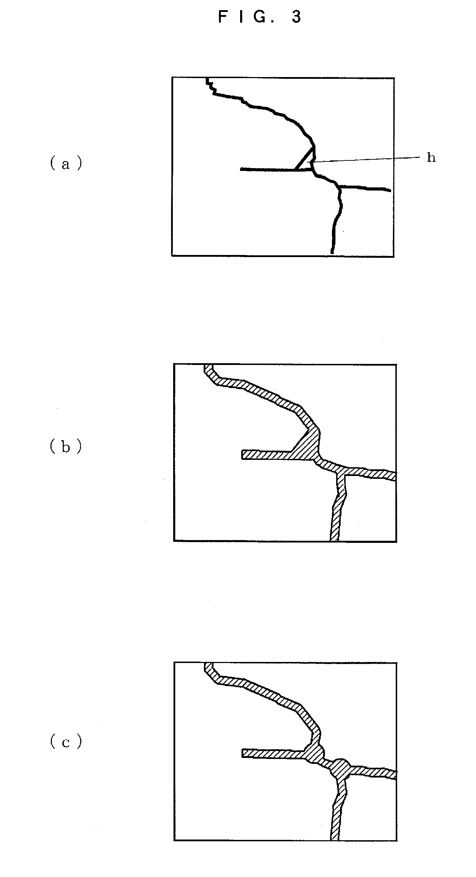

In the case of a living bone, non-interconnecting pores surrounded by a number of cancellous bones or by a cancellous bone and a cortical bone are filled with bone marrow to function appropriately. In the case of an artificial bone, non-interconnecting pores surrounded by beams or by a beam and a wall do not have an inlet for a body fluid, so that they remain hollow after implanted into a living body. When a hollow part h which is likely to become a non-interconnecting pore as shown in Fig. 3(a) is found in the center line setting stage, the artificial bone image data may be formed by filling the hollow part as shown in Fig. 3(b) while drawing a beam or a wall in the image forming step. This improves the mechanical strength. The hollow part to be filled is set on a computer program based on the inner diameter or occlusion rate of the hollow part.

The powder material is typically at least one kind of metal selected from cobalt, tantalum, zirconium, niobium and titanium, and alloys thereof. It may also be a resin such as polylactic acid, polyethylene or polyethylene terephthalate, or a ceramic such as apatite, β-TCP, titanium oxide, bioglass or crystallized glass A-W. An intersection point between the beams may be in part or in whole drawn thicker than the aforementioned uniform diameter as shown in Fig. 3(c) depending on a load expected to be exerted at the time of use. The three-dimensional image data are, for example, STL format data converted from a group of computer tomographic data of a living bone. As for the diameter or thickness of the beam or wall, when the laser spot diameter is denoted by "d", the diameter or thickness of the beam or wall is preferably not less than d and not more than 3d. The diameter or thickness of the beam or wall drawn to be smaller than the laser spot diameter causes the structure of the artificial bone image data not to coincide with the structure of the actual sintered body, and for example, such a case may arise that a interconnecting pore on data is actually formed into a non-interconnecting pore on the sintered body and remains hollow after implantation. On the other hand, when the diameter or thickness of the beam or wall is drawn to be larger than three times the laser spot diameter, the laser should be reciprocated so many times that long time is required for sintering.

When the powder is comprised of a metal, a laser sintered body may be heated at a temperature of 1000°C or higher after the modeling step. This makes particles that are present on the surface of the beam or wall to securely bond to the surface, and neighboring particles to bond to form new micropores.
The method for producing an artificial bone of the present invention is featured by heating the artificial bone precursor thus obtained, after an alkaline treatment when the aforementioned powder is comprised of a metal. This allows formation of a metallic acid salt having an apatite-forming ability on the surface. In order to form a layer of a metal oxide such as anatase on the surface, after an alkaline treatment, dealkalization may be conducted before heating, or heating may be conducted concurrently with dealkalization.
Further, when the powder is able to bond with a bone as is the case of apatite, β-TCP, titanium oxide and the like, the artificial bone precursor itself becomes an artificial bone, whereas when the powder is a resin or another ceramic, the artificial bone precursor itself becomes an artificial bone by conducting laser sintering after mixing it with another powder bondable to bones.

### EFFECT OF THE INVENTION

According to the present invention, since an artificial bone having a structure resembling that of a living bone not only in appearance but also in interior is produced, cells and body fluids are easy to penetrate into the obtained artificial bone, and hence a living bone is easy to be formed there. Furthermore, since it is possible to fill a hollow part and make an intersection point of beams particularly thick, it is possible to realize mechanical strength suited for a person to which the artificial bone is applied.

### BRIEF DESCRIPTION OF THE DRAWINGS

Figs. 1(a) to 1(c) are views showing digital images in each step of the production method of the present invention, in which (a) shows a former half of an extracting step, (b) shows a latter half of the same, and (c) shows an image forming step.
Figs. 2 (a) and 2 (b) depict beams in digital image data, in which (a) is a radial cross section view, and (b) is a cross section view of one layer displayed on the monitor.
Figs. 3 (a) to 3 (c) are views each showing another digital image in each step of the production method of the present invention, in which (a) shows a former half of an extracting step, (b) shows a latter half of the same, and (c) shows an image forming step.
Fig. 4 is a photograph of an artificial bone precursor according to First embodiment.
Fig. 5 is a photograph of an artificial bone precursor according to Second embodiment.
Fig. 6 is a photograph of an artificial bone precursor according to Third embodiment.

### BEST MODES FOR CARRYING OUT THE INVENTION

### First embodiment

A titanium powder having an oxygen content of 0.12% by weight and a maximum particle size of 45 µm produced by a gas atomizing method was prepared.
In parallel with this, a tomogram of a fourth lumbar vertebra of a healthy human being was imaged by using a three-dimensional microcomputer tomography apparatus under the conditions of a tube voltage of 50 kV, a tube current of 40 µA, and a slice thickness of 83.5 µm. DICOM (Digital Imaging and Communications in Medicine) data consisting of about 500 files thus obtained were converted into STL (Stereo Lithography Triangulation Language) format data by a three-dimensional image processing program (TRI/3D-BON available from RATOC), and parts corresponding to a cancellous bone and a cortical bone were extracted. By setting a center line of the extracted part, and drawing a beam and a wall having a diameter or thickness of 0.35 mm at a resolution of 83.5 µm/pixel along the center line, artificial bone image data were generated.

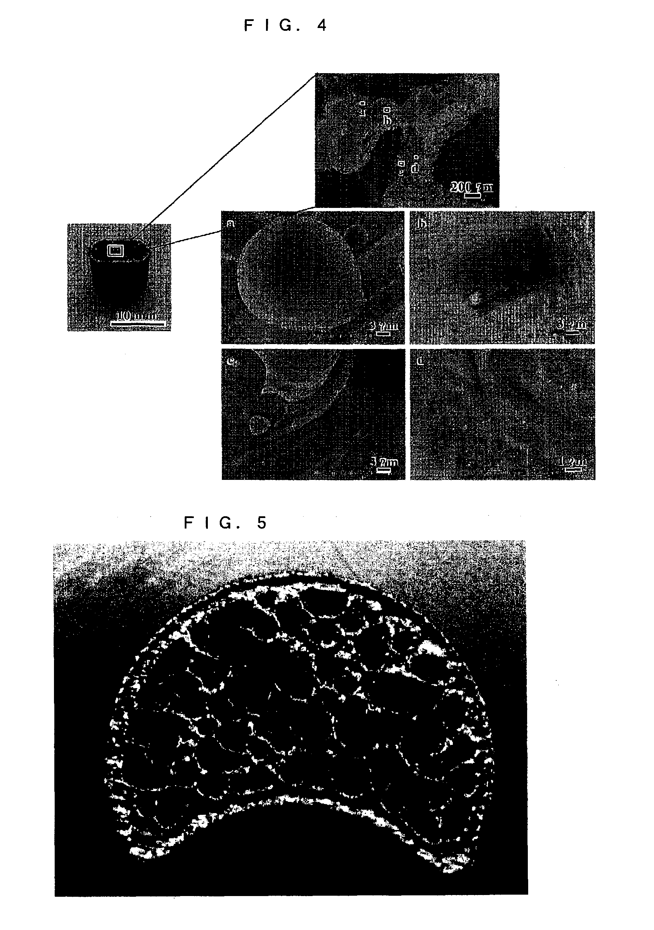

The above titanium powder was charged in a powder chamber of a rapid prototyping apparatus (EOSINT-M270 available from Electro Optical Systems GmbH), and a laser beam was differently set in the following manner for a contour part and a laminate face within the contour part at a thickness of 30 µm per one layer.
Contour part: laser spot diameter 100 µm
Laminate face: laser spot diameter 150 µm
A platform of the apparatus was supplied with the powder for one layer, and irradiated with a Yb fiber laser beam (λ = 1060 to 1100 nm) in an argon atmosphere based on the above artificial bone image data. The platform was descended by a thickness of one layer, and the powder to form the next layer was supplied, and irradiated with a laser beam in a similar manner. By repeating such powder supply and laser irradiation for a required number of times, an artificial bone precursor was produced. A SEM photograph of the obtained artificial bone precursor is shown in Fig. 4.

The artificial bone precursor was immersed in an aqueous solution of 5M sodium hydroxide at 60°C for 24 hours, and immersed in distilled water at 40°C for 48 hours (replaced by fresh distilled water every 12 hours), and then heated at 600°C for one hour, and thus an artificial bone was produced. X-ray diffraction demonstrated that only an anatase phase was formed as a crystal phase on the surface of the artificial bone.
The artificial bone was immersed in a simulated body fluid having substantially the same inorganic ion concentration with a human body fluid. An apatite phase was formed on the surface of the artificial bone after immersion for seven days.

### Second embodiment

Another artificial bone precursor was produced in the same conditions as First embodiment except that a titanium alloy Ti-6Al-4V powder was used in place of the titanium powder in First embodiment. A photograph of the obtained artificial bone precursor is shown in Fig. 5.

### Third embodiment

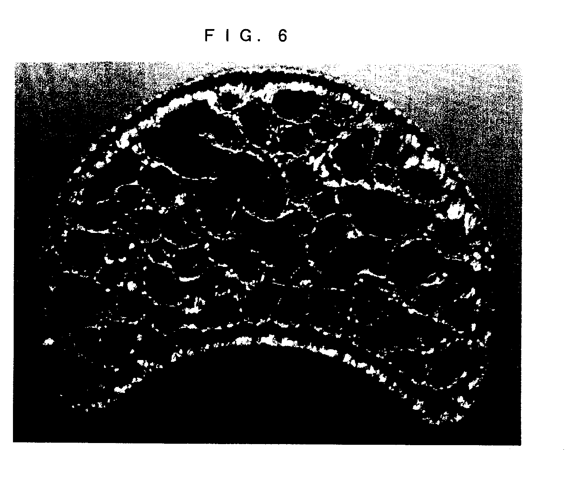

Further another artificial bone precursor was produced in the same conditions as First embodiment except that another titanium alloy Ti-15Mo-5Zr-3Al powder was used in place of the titanium powder in First embodiment. A photograph of the obtained artificial bone precursor is shown in Fig. 6.

## Claims

1. A method for producing an artificial bone precursor, the method comprising:
extracting a part corresponding to a cancellous bone and/or a cortical bone from digitalized three-dimensional image data of a living bone, followed by setting a center line on the part;
drawing a beam or a wall having a uniform diameter or thickness along the center line to form artificial bone image data; and
stacking a sintered layer by laser-sintering a powder of at least one material selected from metals, resins and ceramics based on the artificial bone image data.

2. The method of claim 1, wherein the artificial bone image data is formed by filling a predetermined hollow part during the drawing of the beam or the wall.

3. The method of claim 1 or 2, wherein the powder is a metal powder.

4. The method of claim 3, wherein the metal is at least one selected from the group consisting of cobalt, tantalum, zirconium, niobium and titanium, and alloys thereof.

5. The method of anyone of claims 1 to 4, wherein an intersection point between the beams is in part or in whole drawn thicker than the uniform diameter.

6. The method of anyone of claims 1 to 5, wherein the three-dimensional image data are STL format data converted from a group of computer tomographic data of a living bone.

7. The method of claim 3 or 4, further comprising heating the sintered layer at a temperature of not less than 1000 °C.

8. A method for producing an artificial bone, the method comprising heating the artificial bone precursor obtained according to the method of claim 3 or 4 after an alkaline treatment.

9. The method of claim 8, wherein dealkalization is conducted before heating after the alkaline treatment or concurrently with heating.

10. The method of claim 8 or 9, wherein the precursor is immersed in a simulated body fluid after the heating.
